# EUROPÄISCHE PATENTANMELDUNG

(11) **EP 4 410 778 A1**
(43) Veröffentlichungstag der Anmeldung: **07.08.2024**
(21) Anmeldenummer: 23154635.9
(22) Anmeldetag: 02.02.2023
(51) Int. Cl.: C07D 263/42, C07C 227/18

(54) **VERFAHREN ZUR REINIGUNG VON 2-PHENYL-4-(3-CARBOALKOXYPROPIONYL)-1,3-OXAZOLIN-5-ON SOWIE NEUE KRISTALLINE FORM B VON 2-PHENYL-4-(3-CARBOMETHOXYPROPIONYL)-1,3-OXAZOLIN-5- ON**

(71) Anmelder: Heraeus Precious Metals GmbH & Co. KG, 63450 Hanau (DE)
(72) Erfinder: KUNNARI, Tero, 63450 Hanau (DE); GREIN, Jenin, 63450 Hanau (DE)
(74) Vertreter: Heraeus IP

(57) **Zusammenfassung**

Verfahren zur Reinigung von durch Umsetzung von N-Benzoylglycin mit Bernsteinsäuremonoalkylesterchlorid in 4-Methylpyridin gebildetem und nach Neutralisation mit einem Überschuss an Salzsäure in Form rotgefärbter Kristalle erhältlichem 2-Phenyl-4-(3-carboalkoxypropionyl)-1,3-oxazolin-5-on, dadurch gekennzeichnet, dass man die rotgefärbten Kristalle in einem organischen Lösemittel oder einem Gemisch mehrerer organischer Lösemittel bis zur Entfärbung suspendiert, wobei das oder die organischen Lösemittel ausgewählt sind aus der Gruppe bestehend aus organischen Lösemitteln mit einer Elutionskraft E⁰ im Bereich von 0,40 bis 0,55, und wobei das oder die organischen Lösemittel einen Wassergehalt im Bereich von 1 bis 10 Vol.-% aufweisen.

## Beschreibung

Die Erfindung betrifft ein Verfahren zur Reinigung von durch Umsetzung von N-Benzoylglycin mit Bernsteinsäuremonoalkylesterchlorid in 4-Methylpyridin gebildetem und nach Neutralisation mit einem Überschuss an Salzsäure in Form rotgefärbter Kristalle erhältlichem 2-Phenyl-4-(3-carboalkoxypropionyl)-1,3-oxazolin-5-on. Die Erfindung betrifft auch nach dem erfindungsgemäßen Reinigungsverfahren gereinigtes 2-Phenyl-4-(3-carboalkoxypropionyl)-1,3-oxazolin-5-on sowie eine neue kristalline Form B von 2-Phenyl-4-(3-carbomethoxypropionyl)-1,3-oxazolin-5-on. Die Erfindung betrifft ferner die Verwendung von nach dem erfindungsgemäßen Verfahren gereinigtem 2-Phenyl-4-(3-carboalkoxypropionyl)-1,3-oxazolin-5-on zur Herstellung von 5-Aminolävulinsäurehydrochlorid oder anderer Salze der 5-Aminolävulinsäure.

DE 22 08 800 (A1) offenbart die Herstellung von 5-Aminolävulinsäurehydrochlorid durch Hydrolyse von 2-Phenyl-4-(3-carbomethoxypropionyl)-1,3-oxazolin-5-on in verdünnter Salzsäure. DE 22 08 800 (A1) offenbart auch die Herstellung des Ausgangsprodukts 2-Phenyl-4-(3-carbomethoxypropionyl)-1,3-oxazolin-5-on als rotgefärbte Kristalle durch Umsetzung von N-Benzoylglycin mit Bernsteinsäuremonomethylesterchlorid in 4-Methylpyridin und Überneutralisation mit Salzsäure. Wie sich nunmehr bei Entwicklungsarbeiten der Anmelderin herausgestellt hat, gelingt es nicht, die Rotfärbung der Kristalle mit dem in Beispiel 1 aus DE 22 08 800 (A1) beschriebenen Waschprozess zu beseitigen.

Aufgabe der Erfindung war die Bereitstellung von 2-Phenyl-4-(3-carboalkoxypropionyl)-1,3-oxazolin-5-on mit höherer Reinheit als der von analog DE 22 08 800 (A1) hergestelltem rotgefärbtem kristallinem Material, insbesondere die Bereitstellung von 2-Phenyl-4-(3-carbomethoxypropionyl)-1,3-oxazolin-5-on mit höherer Reinheit als der des gemäß Beispiel 1 aus DE 22 08 800 (A1) erhältlichen rotgefärbten kristallinen Materials.

Wie aus der folgenden Beschreibung hervorgeht, konnte die Anmelderin zur eigenen Überraschung feststellen, dass die die Rotfärbung bewirkenden Verunreinigungen im analog oder gemäß DE 22 08 800 (A1) herstellbaren 2-Phenyl-4-(3-carboalkoxypropionyl)-1,3-oxazolin-5-on, speziell 2-Phenyl-4-(3-carbomethoxypropionyl)-1,3-oxazolin-5-on in überraschend einfacher und effektiver Weise entfernt werden können durch hinreichend lange andauerndes Suspendieren der jeweiligen rotgefärbten Kristalle in feuchtem organischem Lösemittel geeigneter Polarität, genauer gesagt in organischem Lösemittel geeigneter Polarität und mit gezielt eingestelltem Wassergehalt. Wie die Anmelderin zur eigenen Überraschung weiterhin feststellen konnte, können bei besagtem Suspendieren allerdings nicht nur die Verunreinigungen entfernt und somit für die Herstellung von 5-Aminolävulinsäurehydrochlorid oder anderen Salzen der 5-Aminolävulinsäure geeigneteres, weil reineres 2-Phenyl-4-(3-carboalkoxypropionyl)-1,3-oxazolin-5-on gewonnen werden; vielmehr kann beispielsweise die bekannte kristalline Form A besagten gemäß Beispiel 1 aus DE 22 08 800 (A1) erhältlichen rotgefärbten 2-Phenyl-4-(3-carbomethoxypropionyl)-1,3-oxazolin-5-ons im Zuge besagten Suspendierens in eine neue kristalline Form B übergeführt werden.

Die Erfindung betrifft daher ein Verfahren zur Reinigung von durch Umsetzung von N-Benzoylglycin mit Bernsteinsäuremonoalkylesterchlorid in 4-Methylpyridin gebildetem und nach Neutralisation mit einem Überschuss an Salzsäure in Form rotgefärbter Kristalle erhältlichem 2-Phenyl-4-(3-carboalkoxypropionyl)-1,3-oxazolin-5-on, dadurch gekennzeichnet, dass man die rotgefärbten Kristalle in einem organischen Lösemittel oder einem Gemisch mehrerer organischer Lösemittel bis zur Entfärbung suspendiert, wobei das oder die organischen Lösemittel ausgewählt sind aus der Gruppe bestehend aus organischen Lösemitteln mit einer Elutionskraft E⁰ im Bereich von 0,40 bis 0,55, und wobei das oder die organischen Lösemittel einen Wassergehalt im Bereich von 1 bis 10 Vol.-% aufweisen.

Mittels des erfindungsgemäßen Verfahren zu reinigendes und in Form rotgefärbter Kristalle vorliegendes 2-Phenyl-4-(3-carboalkoxypropionyl)-1,3-oxazolin-5-on, bevorzugt 2-Phenyl-4-(3-carbo-C1-C4-alkoxypropionyl)-1,3-oxazolin-5-on und insbesondere bevorzugt 2-Phenyl-4-(3-carbomethoxypropionyl)-1,3-oxazolin-5-on, kann wie schon gesagt analog oder gemäß DE 22 08 800 (A1), insbesondere analog oder gemäß des dortigen Beispiels 1 hergestellt werden. Die mittels HPLC bestimmte Reinheit des jeweiligen in Form rotgefärbter Kristalle vorliegenden 2-Phenyl-4-(3-carboalkoxypropionyl)-1,3-oxazolin-5-ons liegt dabei jeweils beispielsweise im Bereich von 90 bis 95 HPLC-Area%, d.h. regelmäßig nicht höher als 95 HPLC-Area%; die HPLC-Area%-Angabe bezieht sich dabei auf nichtflüchtiges Material, d.h. auf getrocknetes, von organischem Lösemittel befreites Material oder, im Falle noch organischen Lösemittelrest umfassenden Materials, auf den Materialanteil ohne organischen Lösemittelrest. Mit anderen Worten, mittels des erfindungsgemäßen Verfahrens zu reinigendes 2-Phenyl-4-(3-carboalkoxypropionyl)-1,3-oxazolin-5-on (im Folgenden auch als "ursprüngliches 2-Phenyl-4-(3-carboalkoxypropionyl)-1,3-oxazolin-5-on" bezeichnet) muss nicht vollständig getrocknet sein, es kann einen Rest an organischem Lösemittel umfassen, welcher nicht in die Reinheitsbetrachtung einbezogen wird. Ein solcher Anteil organischen Lösemittels kann insbesondere einem der Synthese des 2-Phenyl-4-(3-carboalkoxypropionyl)-1,3-oxazolin-5-ons nachgeordneten Waschschritt entstammen und beispielsweise nach Abnutschen in den gewaschenen jedoch weiterhin nicht vollständig gereinigten rotgefärbten Kristallen verbleiben; ein solcher Restanteil organischen Lösemittels kann beispielsweise im Bereich von bis zu 10 Gramm pro 100 Gramm lösemittelfeuchter rotgefärbter Kristalle betragen.

Die vorerwähnte Reinheitsbestimmung mittels HPLC kann beispielsweise unter Anwendung folgender Parameter durchgeführt werden: Säule (250 × 4,6 mm, RP-18, 5µm), Injektion 5 µL, Fluss 1 mL/min, Säulentemperatur 25°C, UV-Detektion bei 254 nm, Laufmittel A = 0,1% Trifluoressigsäure (TFA) in Wasser, Laufmittel B = 0,1% TFA in Acetonitril, Gradient von 90% A zu 10% A innerhalb 15 min.

Es ist wesentlich, dass das im erfindungsgemäßen Verfahren als flüssiges Suspensionsmedium verwendete organische Lösemittel oder die als flüssiges Suspensionsmedium im verwendeten Gemisch mehrerer organischer Lösemittel enthaltenen organischen Lösemittel ausgewählt sind aus der Gruppe bestehend aus organischen Lösemitteln mit einer Elutionskraft E⁰ im Bereich von 0,40 bis 0,55. Bei der hier erwähnten Elutionskraft E⁰ handelt es sich um einen empirischen Wert entsprechend dem Konzept der dem Fachmann bekannten elutropen Reihe [vgl. W. Trappe; Biochem. Z. 305, 150 (1940); 306, 316 (1940)]. Die elutrope Reihe sortiert organische Lösemittel nach ihrer Elutionswirkung (Elutionsselektivität) bei der Chromatographie, im vorliegenden Fall mit Kieselgel als Adsorbens. Beispiele für solche organische Lösemittel mit einer Elutionskraft E⁰ im Bereich von 0,40 bis 0,54 umfassen Aceton (E⁰= 0,43), Dioxan (E⁰= 0,43), Ethylacetat (E⁰ = 0,45), Tetrahydrofuran (E⁰= 0,48), Acetonitril (E⁰= 0,50). Bevorzugte Beispiele umfassen Aceton, Acetonitril und Ethylacetat.

Es ist außerdem wesentlich, dass das im erfindungsgemäßen Verfahren verwendete organische Lösemittel oder Gemisch mehrerer organischer Lösemittel einen Wassergehalt im Bereich von 1 bis 10 Vol.-%, bevorzugt 2 bis 5 Vol.-% aufweist. Der Wassergehalt ist dabei stets so bemessen, dass das oder die organischen Lösemittel mit dem Wasser kein Zweiphasensystem ausbilden. Der Fachmann spricht in dem Zusammenhang auch von feuchten organischen Lösemitteln im Gegensatz zu wasserfreien organischen Lösemitteln, sogenannten getrockneten organischen Lösemitteln.

Das im erfindungsgemäßen Verfahren stattfindende Suspendieren birgt keine methodischen Besonderheiten und erfolgt beispielsweise im Temperaturbereich von 10 bis 40 °C, bevorzugt 15 bis 30 °C.

Das Suspendieren kann beispielsweise im Verhältnis von 20 bis 100 g 2-Phenyl-4-(3-carboalkoxypropionyl)-1,3-oxazolin-5-on pro Liter organischem Lösemittel, bevorzugt im Verhältnis von 40 bis 60 g 2-Phenyl-4-(3-carboalkoxypropionyl)-1,3-oxazolin-5-on pro Liter organischem Lösemittel erfolgen.

Das Suspendieren kann beispielsweise mittels Rühren und/oder Schütteln bewirkt werden.

Es ist ferner wesentlich, dass das Suspendieren bis zur Entfärbung, d.h. mindestens bis zur Entfärbung der rotgefärbten Kristalle durchgeführt wird; es schadet nicht, wenn das Suspendieren zeitlich darüber hinausreichend durchgeführt wird. Die Dauer des Suspendierens kann beispielsweise im Bereich von 8 bis 24 Stunden liegen. Zu Beginn respektive während des Suspendierens nimmt die flüssige Phase der Suspension eine Rotfärbung an, welche mit fortschreitender Dauer schwächer wird und schließlich verschwindet. Die Entfärbung, d.h. das Erreichen der Entfärbung kann vom Fachmann visuell erkannt werden, beispielsweise daran, dass die Kristalle ihre Rotfärbung verloren haben respektive farblos geworden sind und dass vorzugsweise auch die flüssige Phase ihre Rotfärbung verloren hat, beispielsweise lediglich noch eine schwache Gelbfärbung aufweist; besagte Entfärbung korrespondiert mit entsprechenden HPLC-Messungen mit UV/VIS-Detektion bei 500 bis 600 nm, d.h. im Bereich spezifischer Absorption der die Rotfärbung verursachenden Verunreinigung oder Verunreinigungen. Mit anderen Worten, die ursprünglich in diesem Wellenlängenbereich detektierbaren Signale nehmen im Zuge des visuell wahrnehmbaren Entfärbungsvorgangs ab bis hin zu ihrem Verschwinden.

Führt man das Suspendieren im an sich gleichen, jedoch wasserfreien getrockneten organischen Lösemittel(gemisch) und unter ansonsten gleichen Bedingungen durch, verschwindet die Rotfärbung nicht. Es wird angenommen, dass die die Rotfärbung verursachenden Verunreinigungen während des Suspendierens gemäß dem erfindungsgemäßen Verfahren chemisch verändert, möglicherweise hydrolysiert werden unter Bildung farbloser, im verwendeten organischen Lösemittel(gemisch) löslicher Produkte, während 2-Phenyl-4-(3-carboalkoxypropionyl)-1,3-oxazolin-5-on chemisch unverändert bleibt und nach Beendigung des Suspendierens als Kristalle, genauer gesagt als farblose Kristalle gewonnen werden kann.

Zwecks Gewinnung des gereinigten 2-Phenyl-4-(3-carboalkoxypropionyl)-1,3-oxazolin-5-ons können übliche dem Fachmann bekannte Fest-Flüssig-Trennverfahren angewendet werden, wie Dekantieren, Filtrieren, Abnutschen, Zentrifugieren. Anschließend kann das so abgetrennte noch lösemittelfeuchte 2-Phenyl-4-(3-carboalkoxypropionyl)-1,3-oxazolin-5-on vom organischen Lösemittelrest befreit werden durch Trocknen beispielsweise bei 20 bis 40 °C, gegebenenfalls unterstützt durch verminderten Druck. Das gereinigte 2-Phenyl-4-(3-carboalkoxypropionyl)-1,3-oxazolin-5-on ist farblos oder, anders ausgedrückt, von seiner ursprünglichen Rotfärbung befreit, d.h. es liegt als farblose Kristalle vor.

Mittels des erfindungsgemäßen Verfahrens gereinigtes und getrocknetes 2-Phenyl-4-(3-carboalkoxypropionyl)-1,3-oxazolin-5-on als solches ist chemisch unverändert gegenüber besagtem rotgefärbten noch zu reinigenden 2-Phenyl-4-(3-carboalkoxypropionyl)-1,3-oxazolin-5-on; es umfasst kein Kristallwasser, d.h. es ist kein Hydrat, wie mittels Karl-Fischer-Titration nachgewiesen werden konnte. Seine mittels HPLC bestimmbare Reinheit ist höher als die des rotgefärbten 2-Phenyl-4-(3-carboalkoxypropionyl)-1,3-oxazolin-5-ons und liegt im Allgemeinen im Bereich von >95 bis 99 HPLC-Area%.

Unerwarteterweise weist mittels des erfindungsgemäßen Verfahrens gereinigtes und getrocknetes 2-Phenyl-4-(3-carbomethoxypropionyl)-1,3-oxazolin-5-on eine gegenüber dem ursprünglichen, d.h. noch ungereinigten und rotgefärbten 2-Phenyl-4-(3-carbomethoxypropionyl)-1,3-oxazolin-5-on veränderte kristalline Form B auf, wie sich mittels Röntgenpulverdiffraktometrie (XRPD) nachweisen lässt. So weist das erfindungsgemäß gereinigte und in Gestalt von farblosen stangenförmigen Kristallen vorliegende 2-Phenyl-4-(3-carbomethoxypropionyl)-1,3-oxazolin-5-on (kristalline Form B) ein vom noch zu reinigenden rotgefärbten 2-Phenyl-4-(3-carbomethoxypropionyl)-1,3-oxazolin-5-on abweichendes Pulver-Röntgenbeugungsmuster, das die folgenden Reflexionen umfasst: 7,8, 9,1, 13,3, 16,6, 19,5, 19,7, 20,4, 23,0, 23,6, 23,9, 24,1, 24,5, 25,1, 26,1 und 28,0 ± 0,2° 2Θ. Im Detail weist das erfindungsgemäß gereinigte 2-Phenyl-4-(3-carbomethoxypropionyl)-1,3-oxazolin-5-on folgendes XRPD-Pulverdiffraktogramm^{*)} auf:

| **Peaknummer** | **2θ (°)** | **Intensität (%)** |
|---|---|---|
| 1 | 7,8 | 30,9 |
| 2 | 9,1 | 23,0 |
| 3 | 13,3 | 18,9 |
| 4 | 16,6 | 87,3 |
| 5 | 19,5 | 28,7 |
| 6 | 19,7 | 22,3 |
| 7 | 20,4 | 100,0 |
| 8 | 23,0 | 17,1 |
| 9 | 23,6 | 31,2 |
| 10 | 23,9 | 54,7 |
| 11 | 24,1 | 24,5 |
| 12 | 24,5 | 51,9 |
| 13 | 25,1 | 13,3 |
| 14 | 26,1 | 25,0 |
| 15 | 28,0 | 17,2 |

Zum Vergleich, besagtes rotgefärbtes 2-Phenyl-4-(3-carbomethoxypropionyl)-1,3-oxazolin-5-on der kristallinen Form A zeichnet sich hingegen im Detail durch folgendes XRPD-Pulverdiffraktogramm^{*)} aus:

| **Peaknummer** | **2θ (°)** | **Intensität (%)** |
|---|---|---|
| 1 | 6,2 | 15,4 |
| 2 | 6,4 | 65,5 |
| 3 | 17,6 | 16,1 |
| 4 | 19,1 | 37,6 |
| 5 | 20,2 | 39,7 |
| 6 | 21,9 | 25,9 |
| 7 | 23,5 | 10,0 |
| 8 | 26,6 | 19,1 |
| 9 | 27,0 | 100,0 |
| 10 | 27,8 | 19,4 |

| | | |
|---|---|---|
| ^{*)} Anmerkung: Gezeigt sind jeweils nur die Signale mit einer Intensität von ≥ 10%. Die beiden als Liste dargestellten XRPD-Pulverdiffraktogramme wurden unter gleichen Bedingungen aufgenommen, d.h. jeweils mit einem STOE Powder Diffraction System unter Verwendung von CuKα-Strahlung (40kV, 25mA). Die Genauigkeit der 2Θ-Werte betrug ±0,2°. | | |

Mittels des erfindungsgemäßen Verfahrens gereinigtes 2-Phenyl-4-(3-carboalkoxypropionyl)-1,3-oxazolin-5-on, und insbesondere dabei das 2-Phenyl-4-(3-carbomethoxypropionyl)-1,3-oxazolin-5-on kann zur Herstellung von 5-Aminolävulinsäurehydrochlorid oder anderer Salze der 5-Aminolävulinsäure verwendet werden. Beispielsweise kann es durch Hydrolyse in Gegenwart von Salzsäure zu 5-Aminolävulinsäurehydrochlorid umgesetzt werden, beispielsweise entsprechend der aus DE 22 08 800 (A1) bekannten Herstellungsvorschrift. Vorteilhaft ist dabei, dass die Herstellung des 5-Aminolävulinsäurehydrochlorids oder anderer Salze der 5-Aminolävulinsäure mit einem reineren 2-Phenyl-4-(3-carboalkoxypropionyl)-1,3-oxazolin-5-on-Ausgangsprodukt in Form des erfindungsgemäß gereinigten 2-Phenyl-4-(3-carboalkoxypropionyl)-1,3-oxazolin-5-ons erfolgen kann. Insofern betrifft die vorliegende Erfindung auch dessen Verwendung zur Herstellung von 5-Aminolävulinsäurehydrochlorid durch Hydrolyse in Gegenwart von Salzsäure oder zur Herstellung anderer Salze der 5-Aminolävulinsäure.

Ausführungsbeispiel: Es wurde zunächst wie in Beispiel 1 aus DE 22 08 800 (A1) gearbeitet. Dazu wurden der Lösung von 40 g N-Benzoylglycin in 200 ml 4-Methylpyridin, die in einen mit Thermometer, Rührwerk und Tropftrichter versehenen und durch Eis und Salz abgekühlten Dreihalskolben von 500 mL Inhalt eingetragen worden war, 56 ml Bernsteinsäuremonomethylesterchlorid unter Rühren mit einer solchen Geschwindigkeit zugegeben, dass die Temperatur der Reaktionsmasse im Bereich von -5 bis 0°C lag.

Nach der Beendigung der Säurechloridzugabe rührte man die Reaktionsmasse weitere 5 Stunden bei dieser Temperatur, dann goss man dieselbe in ein Gemisch von 1 kg Eis mit 160 ml konzentrierter Salzsäure unter Rühren aus.

Die ausgefallenen rotgefärbten Kristalle von 2-Phenyl-4-(3-carbomethoxypropionyl)-1,3-oxazolin-5-on (kristalline Form A) wurden abfiltriert.

10 g der rotgefärbten Kristalle wurden mit 45 mL Wasser gewaschen, filtriert und anschließend zweimal mit je 20 mL trockenem Ethylacetat gewaschen. Der Feststoff wurde dann in 20 mL feuchtem Ethylacetat (3 Vol.-% Wasseranteil) suspendiert. Dazu wurde 10 Stunden gerührt, bis die Rotfärbung der Kristalle als auch die Rotfärbung der Ethylacetat-Phase verschwunden war. Die Suspension wurde filtriert und die abfiltrierten stangenförmigen Kristalle von 2-Phenyl-4-(3-carbomethoxypropionyl)-1,3-oxazolin-5-on (kristalline Form B) wurden anschließend einmal mit 20 mL trockenem Ethylacetat gewaschen und getrocknet.

Die Ausbeute betrug 7,1 g, bezogen auf die 10 g rotgefärbten Kristalle.

Mittels HPLC wurde die Reinheit bestimmt unter Anwendung folgender Parameter: Säule (250 x 4,6 mm, RP-18, 5µm), Injektion 5 µL, Fluss 1 mL/min, Säulentemperatur 25°C, UV-Detektion bei 254 nm, Laufmittel A = 0,1% Trifluoressigsäure (TFA) in Wasser, Laufmittel B = 0,1% TFA in Acetonitril, Gradient von 90% A zu 10% A innerhalb 15 min. Die so bestimmte Reinheit betrug 97,2 HPLC-Area%.

Bezüglich der XRPD-Messung wird auf das in der Beschreibung Vorerwähnte verwiesen.

Vergleichsbeispiel 1: Es wurde so gearbeitet wie im Ausführungsbeispiel mit dem Unterschied, dass kein feuchtes Ethylacetat verwendet wurde. Die Rotfärbung war nicht verschwunden.

Vergleichsbeispiel 2: Es wurde so gearbeitet wie im Ausführungsbeispiel mit dem Unterschied, dass nur 1 Stunde gerührt wurde. Die Rotfärbung war noch deutlich sichtbar.

## Patentansprüche

1. Verfahren zur Reinigung von durch Umsetzung von N-Benzoylglycin mit Bernsteinsäuremonoalkylesterchlorid in 4-Methylpyridin gebildetem und nach Neutralisation mit einem Überschuss an Salzsäure in Form rotgefärbter Kristalle erhältlichem 2-Phenyl-4-(3-carboalkoxypropionyl)-1,3-oxazolin-5-on, **dadurch gekennzeichnet, dass** man die rotgefärbten Kristalle in einem organischen Lösemittel oder einem Gemisch mehrerer organischer Lösemittel bis zur Entfärbung suspendiert, wobei das oder die organischen Lösemittel ausgewählt sind aus der Gruppe bestehend aus organischen Lösemitteln mit einer Elutionskraft E⁰ im Bereich von 0,40 bis 0,55, und wobei das oder die organischen Lösemittel einen Wassergehalt im Bereich von 1 bis 10 Vol.-% aufweisen.

2. Verfahren nach Anspruch 1, wobei es sich bei dem Bernsteinsäuremonoalkylesterchlorid um ein Bernsteinsäuremono-C1-C4-alkylesterchlorid und bei dem 2-Phenyl-4-(3-carboalkoxypropionyl)-1,3-oxazolin-5-on um ein 2-Phenyl-4-(3-carbo-C1-C4-alkoxypropionyl)-1,3-oxazolin-5-on handelt.

3. Verfahren nach Anspruch 2, wobei es sich bei dem Bernsteinsäuremono-C1-C4-alkylesterchlorid um Bernsteinsäuremonomethylesterchlorid und bei dem 2-Phenyl-4-(3-carbo-C1-C4-alkoxypropionyl)-1,3-oxazolin-5-on um 2-Phenyl-4-(3-carbomethoxypropionyl)-1,3-oxazolin-5-on handelt.

4. Verfahren nach einem der vorhergehenden Ansprüche, wobei das organische Lösemittel oder Gemisch mehrerer organischer Lösemittel einen Wassergehalt im Bereich von 2 bis 5 Vol.-% aufweist.

5. Verfahren nach einem der vorhergehenden Ansprüche, wobei der Wassergehalt des organischen Lösemittels oder Gemischs mehrerer organischer Lösemittel so bemessen ist, dass das oder die organischen Lösemittel mit dem Wasser kein Zweiphasensystem ausbilden.

6. Verfahren nach einem der vorhergehenden Ansprüche, wobei das Suspendieren im Temperaturbereich von 10 bis 40 °C erfolgt.

7. Verfahren nach einem der vorhergehenden Ansprüche, wobei das Suspendieren im Verhältnis von 20 bis 100 g der rotgefärbten Kristalle pro Liter organischem Lösemittel erfolgt.

8. Verfahren nach einem der vorhergehenden Ansprüche, wobei das Suspendieren mittels Rühren und/oder Schütteln bewirkt wird.

9. Verfahren nach einem der vorhergehenden Ansprüche, wobei man das gereinigte 2-Phenyl-4-(3-carboalkoxypropionyl)-1,3-oxazolin-5-on nach Beendigung des Suspendierens mittels Fest-Flüssig-Trennverfahren als Kristalle abtrennt.

10. Verfahren nach Anspruch 9, wobei die abgetrennten Kristalle getrocknet werden.

11. Gereinigtes 2-Phenyl-4-(3-carboalkoxypropionyl)-1,3-oxazolin-5-on erhältlich nach einem Verfahren eines der Ansprüche 9 oder 10.

12. Gereinigtes 2-Phenyl-4-(3-carboalkoxypropionyl)-1,3-oxazolin-5-on nach Anspruch 11, wobei es sich bei dem 2-Phenyl-4-(3-carboalkoxypropionyl)-1,3-oxazolin-5-on um ein 2-Phenyl-4-(3-carbo-C1-C4-alkoxypropionyl)-1,3-oxazolin-5-on handelt.

13. Gereinigtes 2-Phenyl-4-(3-carbo-C1-C4-alkoxypropionyl)-1,3-oxazolin-5-on nach Anspruch 12, wobei es sich bei dem 2-Phenyl-4-(3-carbo-C1-C4-alkoxypropionyl)-1,3-oxazolin-5-on um 2-Phenyl-4-(3-carbomethoxypropionyl)-1,3-oxazolin-5-on handelt.

14. 2-Phenyl-4-(3-carbomethoxypropionyl)-1,3-oxazolin-5-on in der kristallinen Form B mit einem Pulver-Röntgenbeugungsmuster, das die folgenden Reflexionen umfasst: 7,8, 9,1, 13,3, 16,6, 19,5, 19,7, 20,4, 23,0, 23,6, 23,9, 24,1, 24,5, 25,1, 26,1 und 28,0 ± 0,2° 2Θ.

15. Verwendung eines 2-Phenyl-4-(3-carboalkoxypropionyl)-1,3-oxazolin-5-ons nach einem der Ansprüche 11 bis 14 zur Herstellung von 5-Aminolävulinsäurehydrochlorid durch Hydrolyse in Gegenwart von Salzsäure oder zur Herstellung anderer Salze der 5-Aminolävulinsäure.
